# EUROPEAN PATENT APPLICATION

(11) **EP 2 829 865 A1**
(43) Date of publication of application: **28.01.2015**
(21) Application number: 13764777.2
(22) Date of filing: 01.02.2013
(51) Int. Cl.: G01N 21/64

(54) **CHEMICAL SENSOR, CHEMICAL SENSOR MANUFACTURING METHOD, AND CHEMICAL MATERIAL DETECTING APPARATUS**

(30) Priority: 19.03.2012 JP 2012062855
(71) Applicant: Sony Corporation, Tokyo 108-0075 (JP)
(72) Inventor: MOGI, Hideaki, Tokyo 108-0075 (JP); MATSUZAWA, Nobuyuki, Tokyo 108-0075 (JP); MAEDA, Kensaku, Tokyo 108-0075 (JP); MORIYA, Yusuke, Tokyo 108-0075 (JP)
(74) Representative: MFG Patentanwälte Meyer-Wildhagen Meggle-Freund Gerhard PartG mbB
(86) International application number: PCT/JP2013/000559
(87) International publication number: WO 2013/140707

(57) **Abstract**

[Object] To provide a chemical sensor capable of detecting light emitted from a detection target object efficiently, a method of producing the chemical sensor, and a chemical detection apparatus.

[Solving Means] A chemical sensor according to the present technology includes a substrate and a lens layer. On the substrate, at least one light detection unit is formed. The lens layer is laminated on the substrate and has optical transparency, and a lens structure is formed on a surface of the lens layer opposite to the substrate in a concave shape toward a lamination direction.

## Description

### Technical Field

The present technology relates to a chemical sensor that detects a chemical using light emitted from a detection target object, a method of producing the chemical sensor, and a chemical detection unit.

### Background Art

A chemical sensor that uses emitted light caused due to a chemical bond to detect a chemical has been studied. Specifically, a probe material that specifically binds to a target material to be detected is fixed on a sensor, and a sample is supplied to the sensor. As a result, a target material included in the sample binds to the probe material. For example, if a fluorescent label that can be introduced into combined materials including a target material and a probe material is used to cause the combined materials to emit light, a photoelectric conversion element can detect the emitted light. By fixing a plurality of types of probe materials on the sensor, it is also possible to identify the type of the target material included in the sample.

In order to perform detection with high sensitivity and high accuracy with such a chemical sensor, there is a need to introduce emitted light, which is caused due to binding of a target material and a probe material, into a photoelectric conversion element efficiently. For example, Patent Document 1 discloses a semiconductor device for detecting an organic molecule, in which a solid-state imaging element is integrated with a silicon substrate having an area in which an organic molecule probe is arranged. The device has a configuration in which a solid-state imaging element detects fluorescent light caused due to binding of an organic molecule probe arranged in the organic molecule probe arranging area and a target material.

On the other hand, Patent Document 2 discloses a biopolymer analysis chip in which an on-chip lens is mounted between spots, each of which includes a double-gate transistor (photoelectric conversion element) and a probe material. The chip has a configuration in which fluorescent light generated from combined materials of a probe material and a target material is collected by the on-chip lens, and the double-gate transistor detects the collected light.

### Citation List

### Patent Document

Patent Document 1: Japanese Patent Application Laid-open No. 2002-202303
Patent Document 2: Japanese Patent Application Laid-open No. 2006-4991

### Disclosure of the Invention

### Problem to be solved by the Invention

In the configuration described in Patent Document 1, however, there is no optical system that introduces isotropic emitted light from an organic molecule probe into a solid-state imaging element, which causes a problem in which a sufficient amount of light cannot be obtained and the sensitivity and precision are low. Furthermore, the isotropic emitted light enters an adjacent solid-state imaging element, which may generate cross talk in the detected signal. In addition, the material of the surface that causes an organic molecule probe to bind thereto is not defined, and the improvement of detection accuracy by uniform binding of an organic molecule probe on the surface has not been promoted.

On the other hand, in the configuration described in Patent Document 2, a light transmissive top-gate electrode is formed on the upper surface of an on-chip lens. Such a top-gate electrode is considered to be formed of indium tin oxide (ITO), graphene, or the like, which is a light transmissive electrode material. However, in order to obtain a low resistance value using these materials, there is a need to increase the film thickness, which may lower the light transmittance of the film and the sensitivity.

In view of the circumstances as described above, it is an object of the present technology to provide a chemical sensor capable of detecting light emitted from a detection target object efficiently, a method of producing the chemical sensor, and a chemical detection apparatus.

### Means for solving the Problem

In view of the circumstances as described above, a chemical sensor according to an embodiment of the present technology includes a substrate and a lens layer.

On the substrate, at least one light detection unit is formed.

The lens layer is laminated on the substrate and has optical transparency, and a lens structure is formed on a surface of the lens layer opposite to the substrate in a concave shape toward a lamination direction.

With this configuration, it is possible to cause the detection target object to be collected on the lens structure formed in a concave shape, and to collect, toward the light detection unit, emitted light (fluorescent light, etc.) emitted from the detection target object. Even if the lens layer has any refractive index (absolute refractive index), the refractive index is larger than the refractive index of air around the detection target object. Therefore, the concave lens structure formed on the lens layer functions as a lens. Specifically, with the chemical sensor, it is possible to efficiently detect light emitted from the detection target object.

The lens structure may face the light detection unit.

With this configuration, emitted light is collected toward one light detection unit by one lens structure. Therefore, a corresponding light detection unit can detect light emitted from the detection target object collected on the lens structure.

The light detection unit may include a plurality of light detection units, the plurality of light detection units may be arranged on the substrate, the lens structure may include a plurality of lens structures, and the respective lens structures may face the respective light detection units.

With this configuration, the light detection unit corresponding to each lens structure can detect light emitted from the detection target object contained in the lens structure. In addition, by the light collection effects of the lens structure, a light detection unit is prevented from detecting light emitted from an adjacent lens structure (cross talk).

The lens layer may have shielding properties with respect to a wavelength band of illumination light applied to the chemical sensor.

With this configuration, illumination light (excitation light, etc.) for causing the detection target object to generate emitted light can be shielded by the lens layer, the light detection unit can be prevented from detecting the illumination light, and the light detection unit can detect only the emitted light.

The chemical sensor may include a spectral filter layer that is laminated between the substrate and the lens layer, and has shielding properties with respect to a wavelength band of illumination light applied to the chemical sensor.

With this configuration, illumination light can be shielded by the spectral filter layer, the light detection unit can be prevented from detecting the illumination light, and the light detection unit can detect only the emitted light.

The lens layer may have a first refractive index and the spectral filter layer may have a second refractive index that is larger than the first refractive index.

With this configuration, because emitted light refracted by the lens layer is further refracted on the interface between the lens layer and the spectral filter layer, the light detection unit can collect emitted light more.

It may include a protective layer that is laminated on the lens layer and has liquid-repellent properties with respect to a solution containing a detection target object.

With this configuration, a detection target object-containing solution supplied to the chemical sensor has a large contact angle with the protective layer due to the liquid-repellent properties of the protective layer. Therefore, if the detection target object-containing solution is dried gradually, the detection target object-containing solution is collected on the lens structure by the concave shape of the lens structure and the liquid-repellent properties. Specifically, it is possible to collect the detection target object on the lens structure, and to increase the light collection effects of the lens structure.

The surface of the lens layer opposite to the substrate may have liquid-repellent properties with respect to a solution containing a detection target object.

With this configuration, it is possible to collect the detection target object-containing solution supplied to the chemical sensor on each lens structure by the liquid-repellent properties of the lens layer and the concave shape of the lens structure.

The lens structure may be formed in a spherical lens shape.

With this configuration, it is possible to express not only the light collection effects by the refractive index of the lens layer (first refractive index) but also the light collection effects by the shape of the lens.

The lens structure may be formed in a cylindrical lens shape.

With this configuration, it is possible to express not only the light collection effects by the refractive index of the lens layer (first refractive index) but also the light collection effects by the shape of the lens.

In view of the circumstances as described above, a method of producing a chemical sensor according to an embodiment of the present technology includes laminating a thermoplastic material on a substrate on which a plurality of light detection units are formed.

The thermoplastic material is patterned into a plurality of sections.

The adjacent sections of the thermoplastic material are connected to each other by heating the thermoplastic material, and the thermoplastic material is formed in a concave shape.

With this configuration, it is possible to form the thermoplastic material in a concave shape by the fluidity of the thermoplastic material only by patterning and heating the thermoplastic material. The thermoplastic material may be used as a lens layer, or an etching resist for forming a lens layer. Specifically, with this producing method, it is possible to produce the chemical sensor having a concave lens structure easily.

In the process of patterning the thermoplastic material, the thermoplastic material may be patterned by removing the thermoplastic material above the light detection unit linearly.

With this configuration, because the thermoplastic material above each light detection unit is removed, a portion where the thermoplastic material is thinly connected above each light detection unit is formed when the thermoplastic material is heated, i.e., it is possible to form the lens structure facing each light detection unit.

In view of the circumstances as described above, a chemical detection apparatus according to an embodiment of the present technology includes a chemical sensor, an illumination light source, and an image acquisition unit.

The chemical sensor includes a substrate on which at least one light detection unit is formed, a lens layer that is laminated on the substrate and has optical transparency, and a lens structure formed on a surface of the lens layer opposite to the substrate in a concave shape toward a lamination direction.

The illumination light source applies illumination light to the chemical sensor.

The image acquisition unit acquires, based on an output from the light detection unit generated by emitted light that is generated from a detection target object contained in the lens structure by irradiation of the illumination light, an image of the emitted light.

With this configuration, it is possible to apply illumination light to the chemical sensor, to acquire, based on an output from the light detection unit generated by emitted light that is generated from the detection target object by irradiation of the illumination light, an image of the emitted light, and to detect a chemical contained in each lens structure from the image.

### Effect of the Invention

As described above, according to the present technology, it is possible to provide a chemical sensor capable of detecting light emitted from a detection target object efficiently, a method of producing the chemical sensor, and a chemical detection apparatus. Brief Description of Drawings

[Fig. 1] A plan view of a chemical sensor according to a first embodiment of the present technology.
[Fig. 2] A cross-sectional view of the chemical sensor.
[Fig. 3] A cross-sectional view of the chemical sensor.
[Fig. 4] A schematic diagram showing a chemical detection apparatus according to the first embodiment of the present technology.
[Fig. 5] A measurement flowchart for detecting a chemical using the chemical sensor.
[Figs. 6] Schematic diagrams showing a state in the measurement flow of the chemical sensor.
[Fig. 7] A schematic diagram showing a state where illumination light is applied to the chemical sensor.
[Fig. 8] A schematic diagram showing a state where emitted light is collected by a lens structure in the chemical sensor.
[Fig. 9] A flowchart showing a method of producing the chemical sensor.
[Figs. 10] Plan views showing a state of the chemical sensor at each producing stage.
[Figs. 11] Cross-sectional views showing a state of the chemical sensor at each producing stage.
[Fig. 12] A flowchart showing another method of producing the chemical sensor.
[Fig. 13] A cross-sectional view of a chemical sensor according to a second embodiment of the present technology.
[Fig. 14] A flowchart showing a method of producing the chemical sensor.
[Figs. 15] Cross-sectional views showing a state of the chemical sensor at each producing stage.
[Fig. 16] A perspective view showing a shape and arrangement of the chemical sensor according to the first embodiment of the present technology.
[Fig. 17] A perspective view showing a shape and arrangement of a lens structure in the chemical sensor.
[Fig. 18] A perspective view showing a shape and arrangement of a lens structure in the chemical sensor.

### Mode(s) for Carrying Out the Invention

### (First embodiment)

A chemical detection apparatus according to a first embodiment of the present technology will be described.

### [Whole configuration of chemical sensor]

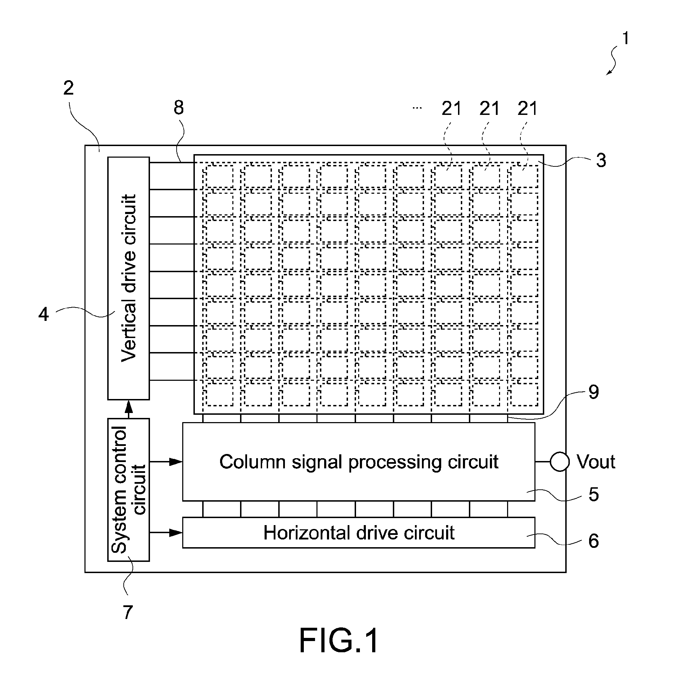

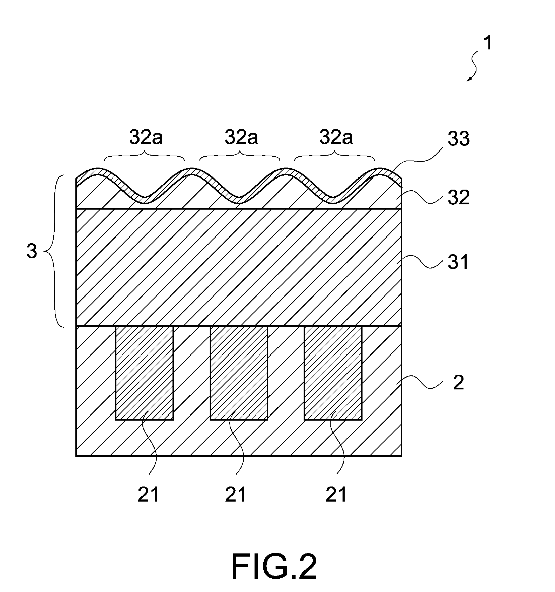

Fig. 1 is a plan view of a chemical sensor 1 according to this embodiment, and Fig. 2 is a cross-sectional view of the chemical sensor 1. As shown in these figures, the chemical sensor 1 includes a light guide unit 3 provided on a substrate 2 and peripheral circuits provided on the substrate 2. A plurality of light detection units 21 are arranged on the substrate 2, and the light guide unit 3 is arranged on the plurality of light detection units 21.

Although the details will be described later, the light guide unit 3 supports a probe material and a target material (detection target object) included in a supplied sample binds to the probe material. Light (fluorescent light, etc.) generated from combined materials including a target material and a probe material enters the light detection unit 21 provided on the substrate 2, and is output as an electric signal by peripheral circuits. Examples of the probe material include DNA (deoxyribonucleic acid), RNA (ribo nucleic acid), a protein, and an antigenic substance, and examples of the target material include chemicals that can bind to these materials.

The light detection unit 21 may be a photoelectric conversion element (photodiode) that coverts light into current. The light detection unit 21 may be an impurity area formed by introducing an impurity on the substrate 2 being a semiconductor substrate. The light detection unit 21 may be connected to a pixel circuit including a gate insulating film and a gate electrode, which are not shown, and the pixel circuit may be provided on a surface of the substrate 2 opposite to the main surface of the substrate 2. The number and arrangement of the light detection units 21 are not limited, and the light detection units 21 may be arranged in a matrix shape or a line shape. Here, the light detection units 21 are arranged on a flat surface of the substrate 2 in a matrix shape, and the direction of the row is assumed to be a vertical direction and the direction of the column is assumed to be a horizontal direction.

The peripheral circuits include a vertical drive circuit 4, a column signal processing circuit 5, a horizontal drive circuit 6, and a system control circuit 7. In addition, each light detection unit 21 is connected to a pixel drive line 8 for each row, and connected to a vertical signal line 9 for each column. Each pixel drive line 8 is connected to the vertical drive circuit 4, and the vertical signal line 9 is connected to the column signal processing circuit 5.

The column signal processing circuit 5 is connected to the horizontal drive circuit 6, and the system control circuit 7 is connected to the vertical drive circuit 4, the column signal processing circuit 5, and the horizontal drive circuit 6. It should be noted that the peripheral circuit may be located of a position of being laminated on a pixel area or arranged on the opposite side of the substrate 2, for example.

The vertical drive circuit 4 includes a shift register, for example, selects the pixel drive line 8, supplies a pulse for driving the light detection unit 21 to the selected pixel drive line 8, and drives the plurality of light detection units 21 row by row. Specifically, the vertical drive circuit 4 selects and scans each light detection units 21 row by row in a vertical direction sequentially. Then, the vertical drive circuit 4 supplies a pixel signal based on a signal charge generated depending on the amount of received light in each light detection units 21 to the column signal processing circuit 5 through the vertical signal line 9 wired vertically to the pixel drive line 8.

The column signal processing circuit 5 performs, for each pixel column, a signal process such as noise removal on a signal output from a row of light detection units 21. Specifically, the column signal processing circuit 5 performs a signal processing such as correlated double sampling (CDS) for removing unique fixed pattern noise of a pixel, a signal amplification, and analog/digital conversion (AD).

The horizontal drive circuit 6 includes a shift register, for example, and outputs a horizontal scanning pulse sequentially, thereby selecting each column signal processing circuit 5 in order and causing each column signal processing circuit 5 to output a pixel signal.

The system control circuit 7 receives an input clock and data that designates an operation mode and the like, and outputs data such as internal information of the light detection unit 21. Specifically, the system control circuit 7 generates, based on a vertical synchronous signal, a horizontal synchronous signal, and a master clock, a clock signal or control signal, which is an operation standard of the vertical drive circuit 4, the column signal processing circuit 5, the horizontal drive circuit 6, and the like. Then, the system control circuit 7 inputs these signals to the vertical drive circuit 4, the column signal processing circuit 5, the horizontal drive circuit 6, and the like.

As described above, the vertical drive circuit 4, the column signal processing circuit 5, the horizontal drive circuit 6, the system control circuit 7, and the pixel circuit provided on the light detection units 21 form the configuration in which light that has entered each light detection unit 21 is output as an electric signal. Such a circuit configuration is only an example, and this embodiment can be applied to any circuit configuration as long as light that has entered the light detection unit 21 can be output.

### [Structure of chemical sensor]

As shown in Fig. 2, the light guide unit 3 is laminated on the substrate 2 on which the light detection units 21 are formed, thereby forming the chemical sensor 1. The light guide unit 3 includes a spectral filter layer 31, a lens layer 32, and a protective layer 33.

The spectral filter layer 31 is laminated on the substrate 2, and disperses light transmitted through the lens layer 32. Although the details will be described later, the spectral filter layer 31 shields the wavelength band of illumination light (excitation light, etc.) applied to the chemical sensor 1, and causes emitted light (fluorescent light, etc.), which is emitted from a detection target object by irradiation of the illumination light, to transmit therethrough. The material of the spectral filter layer 31 is not particularly limited as long as it has such spectral properties. However, it favorably has a large refractive index (second refractive index). The spectral filter layer 31 needs to have a thickness that can exert predetermined spectral properties. However, it is favorable to have a thickness as thin as possible to prevent emitted light from diffusing.

The lens layer 32 is laminated on the spectral filter layer 31, provides a concave lens structure (to be described later) that supports a detection target object, and collects light emitted from the detection target object on the light detection unit 21. The lens layer 32 may be formed of a material that causes emitted light in the wavelength band to be transmitted therethrough and has a predetermined refractive index (first refractive index). It is favorable that the first refractive index is large in order to increase the light collection effects of the lens layer 32. It should be noted that the lens layer 32 does not necessarily have a uniform thickness.

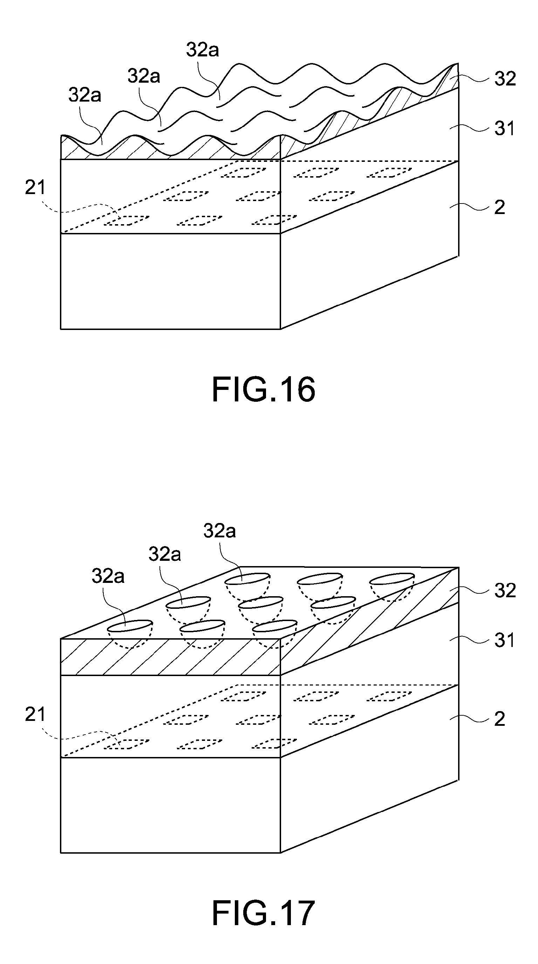

In the lens layer 32, a concave lens structure 32a is formed on the surface opposite to the substrate 2 toward the lamination direction of the lens layer 32. Fig. 16 is a perspective view showing a shape and arrangement of the lens structure 32a. It should be noted that illustration of the protective layer 33 is omitted in Fig. 16. As shown in the figure, the lens structure 32a may have a concave structure formed by a wavelike structure formed on the surface of the lens layer 32.

Because the lens layer 32 has the first refractive index as described above, the lens structure 32a functions as a lens. As shown in Fig. 2 and Fig. 16, a plurality of lens structures 32a may be formed, and each lens structure 32a may be formed to face the light detection unit 21.

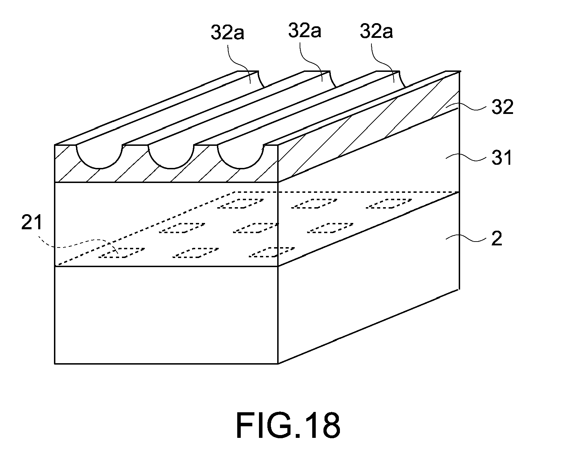

In addition, the shape of the lens structure 32a is not limited to the one shown in Fig. 16. The lens structure 32a may have a concave structure of another shape. Fig. 17 and Fig. 18 are each a perspective view showing a shape and arrangement of the lens structure 32a having another shape. It should be noted that illustration of the protective layer 33 is omitted in Fig. 17 and Fig. 18.

For example, as shown in Fig. 17, the lens structure 32a may have a hemispherical concave shape. In this case, each lens structure 32a forms a spherical lens. In addition, each lens structure 32a does not necessarily face each light detection unit 21 on a one-to-one basis, and one lens structure 32a may face the plurality of light detection units 21. For example, as shown in Fig. 18, the lens structure 32a may have a concave (groove-like) structure of a semicylindrical shape that faces a column of the light detection units 21. In this case, each lens structure 32a forms a cylindrical lens (cylindrical structure).

Because the lens structure 32a functions as a lens as described above, it is favorable that the lens structure 32a has a shape nearer to a lens for the reason that light can be collected on the light detection unit 21 effectively. However, each lens structure 32a functions not only as a lens but also to hold a detection target object at a position that faces the light detection unit 21. Therefore, the lens structure 32a only needs to have a concave structure toward the lamination direction of the lens layer 32, and the shape and arrangement of the lens structure 32a are not particularly limited.
For example, the lens structures 32a may each have a pyramidal concave shape such as a circular cone, a triangular pyramid, and a quadrangular pyramid, which face each light detection unit 21a, or various kinds of groove-like shapes that face each column of the light detection units 21a. In addition, the lens structure 32a does not necessarily need to have the same shape, and may have different shapes.

Specifically, because the lens structure 32a has a concave shape, each lens structure can contain a detection target object. In addition, because the lens layer 32 has the first refractive index, it is possible to collect light emitted from the detection target object on the light detection unit 21. Furthermore, because the lens structure 32a has a shape nearer to a lens, it is possible to obtain light collection effects by the shape. In addition, each lens structure 32a is arranged to face each light detection unit 21 (on a one-to-one basis). Accordingly, it is possible to collect light emitted from a detection target object contained in each lens structure 32a on one light detection unit 21 and to detect emitted light more efficiently.

The lens structure 32a can be formed by a producing method to be described later. In this case, the lens layer 32 may be formed of a thermoplastic material that is softened by heating. On the other hand, the lens structure 32a does not necessarily need to be formed by a heating process. In this case, the lens layer 32 does not necessarily need to be formed of a thermoplastic material.

The protective layer 33 covers the lens layer 32, and holds a detection target object on a surface thereof. As shown in Fig. 2, the protective layer 33 may be thin and formed along the lens structure 32a formed on the surface of the lens layer 32. The protective layer 33 may be formed of a material that causes at least emitted light emitted from a detection target object to transmit therethrough. The protective layer 33 may have a refractive index smaller than the refractive index of the lens layer 32 (first refractive index). However, the protective layer 33 may be thinner than the lens layer 32, and the refractive index provides a small influence thereon. Therefore, the protective layer 33 may have a refractive index larger than the first refractive index.

The surface of the protective layer 33 may have liquid-repellent properties with respect to a solution containing a detection target object (detection target object-containing solution). Although described later, because a detection target object is contained in liquid and is supplied to the surface of the protective layer 33, the protective layer 33 has liquid-repellent properties with respect to the detection target object-containing solution, thereby collecting the detection target object on each lens structure 32a. The liquid-repellent properties represent to be in contact with the detection target object-containing solution at a large contact angle, i.e., to repel the detection target object-containing solution. Specifically, the protective layer 33 may be hydrophobic if the detection target object-containing solution is a hydrophilic liquid, and may be hydrophilic if the detection target object-containing solution is a lipophilic liquid.

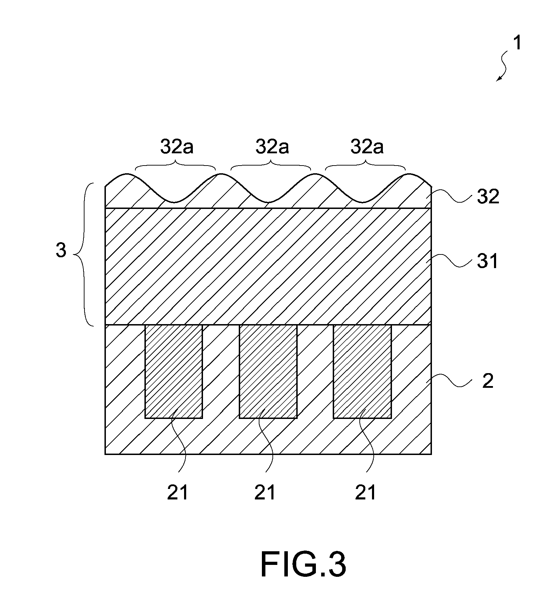

It should be noted that as shown in Fig. 3, the chemical sensor 1 does not necessarily include the protective layer 33. In this case, it is possible to collect the detection target object on each lens structure 32a by applying such a surface process that the surface of the lens layer 32 is made to have liquid-repellent properties with respect to the detection target object-containing liquid to the surface of the lens layer 32. On the other hand, in the case where the process for giving liquid-repellent properties is not applied to the surface of the lens layer 32, the detection target object cannot be collected by liquid-repellent properties. However, it is possible to collect the detection target object on each lens structure 32a to some extent by the concave shape of the lens structure 32a.

### [Configuration of chemical detection apparatus]

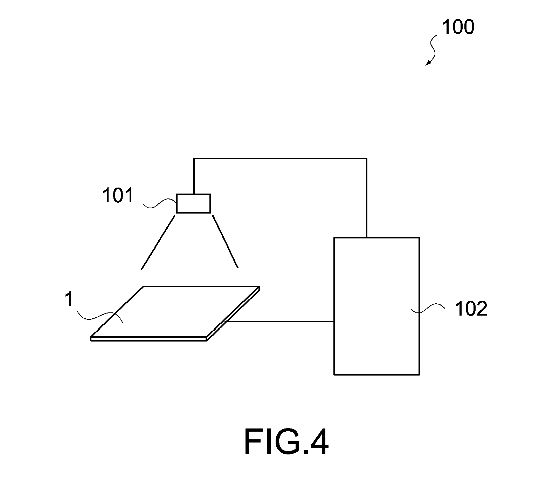

The configuration of a chemical detection apparatus 100 that uses the chemical sensor 1 having the configuration described above to detect a chemical will be described. Fig. 4 is a schematic diagram showing the configuration of the chemical detection apparatus 100. As shown in Fig. 4, the chemical detection apparatus 100 includes the chemical sensor 1, an illumination light source 101, and an image acquisition unit 102.

The illumination light source 101 applies illumination light to the chemical sensor 1. The illumination light may be, for example, excitation light for causing a fluorescent label, which is included in a detection target object held in the chemical sensor 1, to emit fluorescent light. The illumination conditions such as a wavelength and an intensity of illumination light may be appropriately set depending on the type of the fluorescent label and the like.

The image acquisition unit 102 acquires an image of emitted light from an output of the light detection units 21, which is generated from light (fluorescent light, etc.) emitted from a detection target object by illumination light applied from the illumination light source 101. The image acquisition unit 102 may be connected to the column signal processing circuit 5 of the chemical sensor 1, and may acquire an output of each light detection unit 21.

The chemical detection apparatus 100 may have the configuration described above. The configuration of the chemical detection apparatus 100 is only an example, and an apparatus having a different configuration may user the chemical sensor 1.

### [Measurement flow and action of chemical sensor]

The measurement flow for detecting a chemical using the chemical sensor and the action of the chemical sensor 1 at this time will be described.

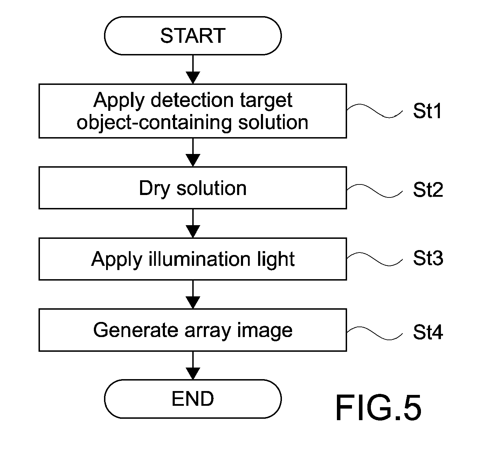

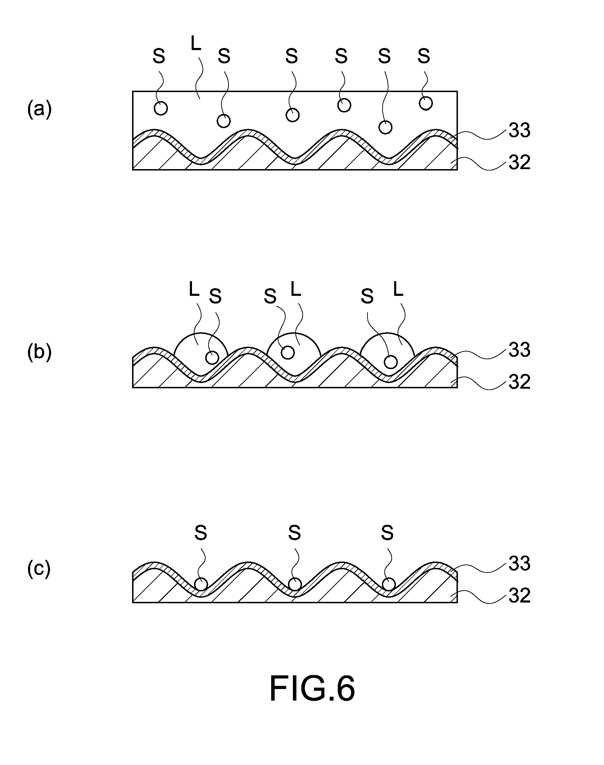

Fig. 5 is a measurement flow for detecting a chemical, and Figs. 6 are schematic diagrams showing a state of the chemical sensor 1. As shown in Fig. 5, a detection target object-containing solution is applied to the light guide unit 3 of the chemical sensor 1 first (St1). It should be noted that a probe material (not shown) that specifically binds to a detection target object may be fixed on each lens structure 32a on the surface of the light guide unit 3. By using a different probe material for each lens structure 32a, a different detection target object (target material) can be bound in each lens structure 32a.

As shown in Fig. 6(a), a detection target containing solution L is supplied to the light guide unit 3 of the chemical sensor 1. The detection target containing solution L contains a detection target object S. If the detection target containing solution L is dried (St2), the detection target containing solution L is collected on the lens structure 32a as shown in Fig. 6(b). This is caused due to the concave shape of the lens structure 32a and the liquid-repellent properties of the protective layer 33 with respect to the detection target containing solution L. In particular, by the liquid-repellent properties of the protective layer 33, the detection target containing solution L is easily collected on the lens structure 32a with surface tension. If the drying of the detection target containing solution L is finished, the detection target object S is attached to the protective layer 33 in the form where it is contained in each lens structure 32a as shown in Fig. 6(c).

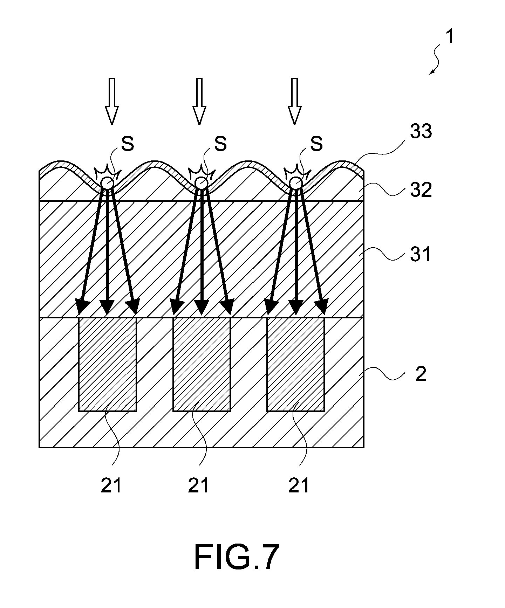

Next, illumination light is applied from the illumination light source 101 to the chemical sensor 1 (St3). Fig. 7 is a schematic diagram showing a state of the chemical sensor 1 irradiated with illumination light. As shown in the figure, illumination light (white arrow) is applied to the detection target object S, and emitted light (black arrow) is generated from the detection target object S. The illumination light may be, for example, excitation light, and the emitted light may be, for example, fluorescent light. It should be noted that substance that generates emitted light (e.g., fluorescent label) may be introduced into the detection target object S in advance, or may be introduced into combined materials of a probe material and a target material (detection target object S).

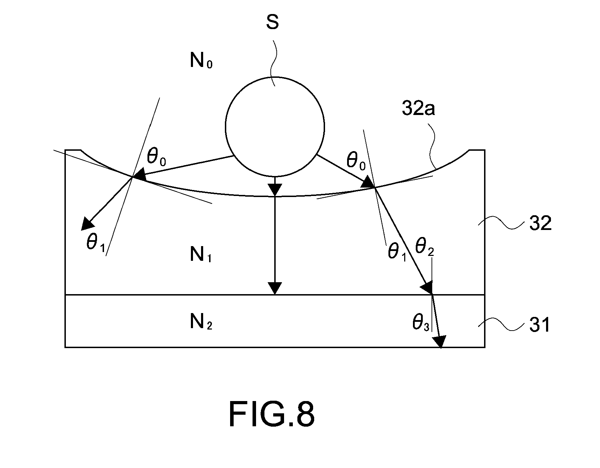

Fig. 8 is a schematic diagram showing a state where emitted light is collected by the lens structure 32a. It should be noted that although the protective layer 33 is omitted in Fig. 8, it is possible to ignore the optical influence of the protective layer 33 because it is thin as descried above.

As shown in Fig. 8, emitted light generated from the detection target object S is refracted on the surface of the lens structure 32a (hereinafter, referred to as lens surface). This is because an incidence angle θ₀ to a lens surface is larger than an output angle θ₁ from the lens surface by the Snell's law because a refractive index (first refractive index) N₁ of the lens layer 32 is larger than a refractive index No of the side of the inspection target S (air). The refraction on the lens surface acts so that diffusion of emitted light converges, i.e., the emitted light is collected toward the light detection unit 21 by the lens surface.

Furthermore, by making a refractive index (second refractive index) N₂ of the spectral filter layer 31 larger than the first refractive index N₁, refraction is caused also on the interface between the lens layer 32 and the spectral filter layer 31. Specifically, an output angle θ3 from the interface is smaller than the incidence angle θ2 to the interface, thereby collecting emitted light toward the light detection unit 21.

Emitted light that has entered the spectral filter layer 31 reaches the light detection unit 21, and is detected (photoelectrically converted) by the light detection unit 21. Because emitted light is collected on the lens surface and the interface between the lens layer 32 and the spectral filter layer 31 as described above, the proportion of emitted light that reaches the light detection unit 21 is increased, and the detection accuracy is prevented from decreasing due to leakage (cross talk) of emitted light to adjacent light detection unit 21.

The illumination light applied to the chemical sensor 1 is shielded by the spectral filter layer 31, and is prevented from being detected by the light detection unit 21. The output of each light detection unit 21 is output to the image acquisition unit 102 via the peripheral circuit, and an array image of emitted light (image that represents an intensity for each light detection unit 21) is generated in the image acquisition unit 102 (St4). By fixing a different probe material on each lens structure 32a as described above, it is possible to identify the type of a detection target object from a position of the light detection unit 21 that has detected emitted light.

As described above, in the chemical sensor 1 according to this embodiment, because the concave lens structure 32a is formed on the lens layer 32, a detection target object is collected on the lens structure 32a, and is arranged at a position that is easily detected by the light detection unit 21. Furthermore, by the lens effects of the lens structure 32a, light emitted from a detection target object is collected on the light detection unit 21, and it is possible to detect the emitted light efficiently.

### [Method of producing chemical sensor]

A method of producing the chemical sensor 1 will be described.

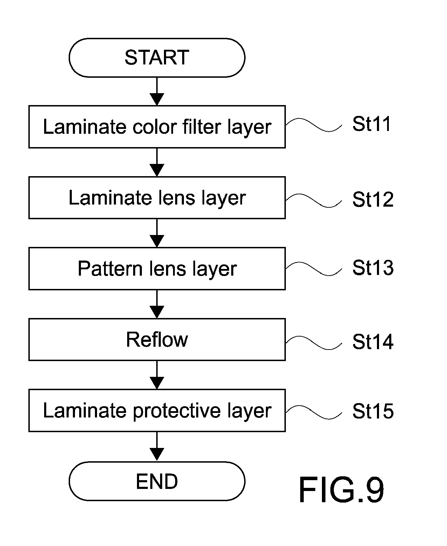

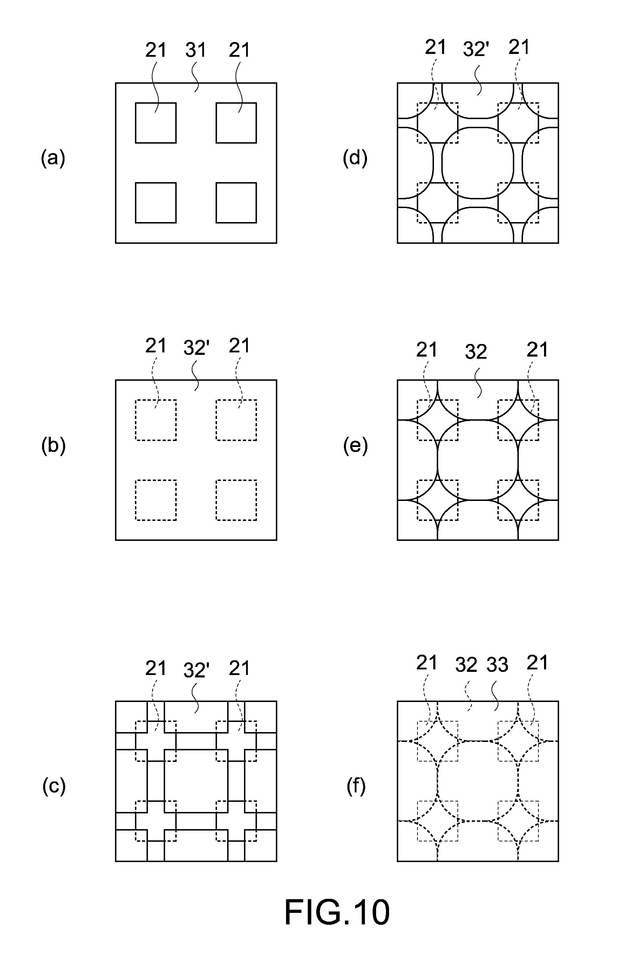

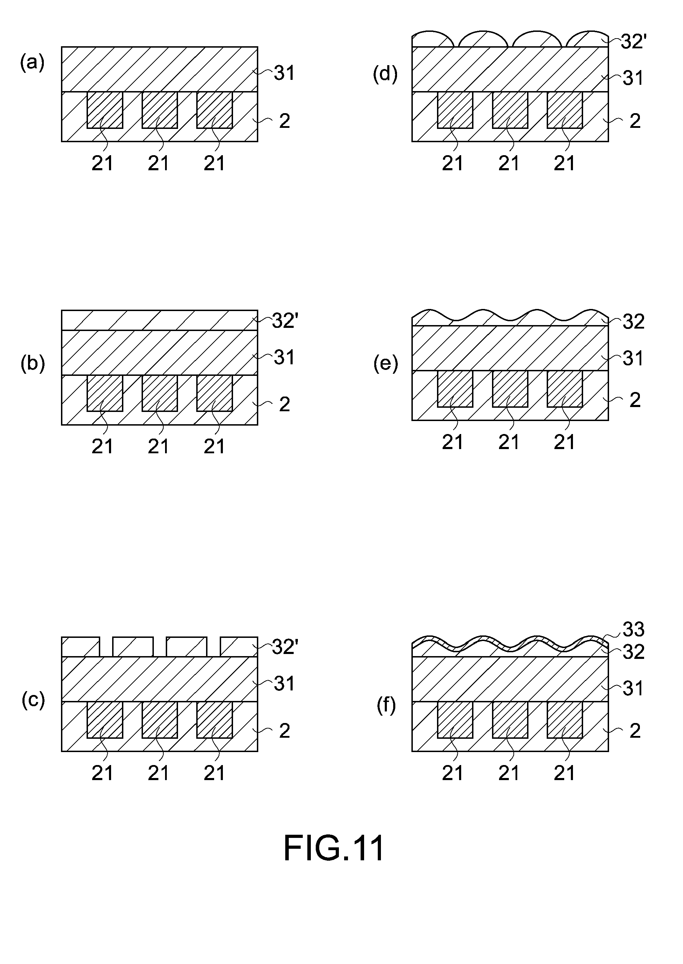

Fig. 9 is a flowchart showing a method of producing the chemical sensor 1. Figs. 10 and Figs. 11 are each schematic diagrams showing the chemical sensor 1 at each producing stage. Figs. 10 are plan views of the chemical sensor 1, and Figs. 11 are cross-sectional views of the chemical sensor 1.

As shown in Fig. 10(a) and Fig. 11(a), on the substrate 2 on which the light detection units 21 are formed, the spectral filter layer 31 is laminated (St11). It is possible to laminate the spectral filter layer 31 by an arbitrary method. Next, as shown in Fig. 10(b) and Fig. 11(b), on the spectral filter layer 31, a lens layer 32' formed of a lens material is laminated (St12). The lens layer 32' can also be laminated by an arbitrary method.

Next, as shown in Fig. 10(c) and Fig. 11(C), the lens layer 32' is patterned (St13). In the patterning of the lens layer 32', the lens layer 32' is divided into a plurality of sections by removing a part of the lens layer 32'. The patterning of the lens layer 32' can be performed by, for example, a lithography technique. It should be noted that as shown in Fig. 10(c), the patterning of the lens layer 32' can be performed by removing the lens layer 32' above each light detection unit 21 linearly. In the case where the plurality of light detection units 21 are arranged in a matrix shape, lines above the light detection units 21 have a lattice shape.

Next, as shown in Fig. 10(d) and Fig. 11(d), the patterned lens layer 32' is heated (reflowed) (St14). By heating, the lens layer 32' becomes a fluid having viscosity. If the heating of lens layer 32' is continued, the viscosity of the lens material is decreased, and it flows. As a result, as shown in Fig. 10(e) and Fig. 11(e), adjacent sections of the lens layer 32' are connected to each other. Because the connected portion is thin, the concave lens structure 32a is formed. If the heating time period is too long, the lens layer 32' becomes flat. Therefore, the heating time period is adjusted so that the lens structure 32a is formed taking into account the fluidity of the lens material.

In the above-mentioned patterning (St13), because the lens layer 32' above each light detection unit 21 is removed linearly, the lens structure 32a in which the lens layer 32' is thin above the intersection point of the lines, i.e., right above each light detection unit 21, is formed. By removing the lens layer 32' above each light detection unit 21 linearly as described above, it is possible to form the lens layer 32 having the lens structure 32a that faces each light detection unit 21 only by heating the lens layer 32'.

Next, as shown in Fig. 10(f) and Fig. 11(f), the protective layer 33 is laminated on the lens layer 32 (St15). It is possible to laminate the protective layer 33 by an arbitrary method. Moreover, the producing process may be finished without laminating the protective layer 33, and such a surface process that the lens layer 32 is made to have liquid-repellent properties with respect to a detection target object-containing solution may be applied to the lens layer 32 instead of the protective layer 33.

Furthermore, the peripheral circuits such as the vertical drive circuit 4 and the column signal processing circuit 5 are mounted on the substrate 2 as necessary, thereby producing the chemical sensor 1. It should be noted that these peripheral circuits may be mounted on the substrate 2 prior to the above-mentioned producing process.

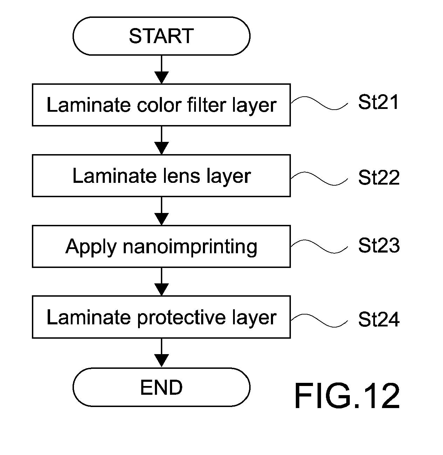

It should be noted that it is also possible to produce the chemical sensor 1 in the following way. Fig. 12 is a flowchart showing another method of producing the chemical sensor 1.

The step of laminating the spectral filter layer 31 on the substrate 2 on which the plurality of light detection units 21 are formed (St21) and the step of laminating the lens layer 32' on the spectral filter layer 31 (St22) are the same as those described above. Next, nanoimprinting is applied to the lens layer 32' (St23).

The nanoimprinting can be performed by pressing a mold of the lens structure 32a on the lens layer 32' that is softened by heating. The position of the mold of the lens structure 32a is adjusted so that the lens structure 32a is formed at a position that faces the plurality of light detection unit 21. The lens layer 32 may be formed in this way. As described above, it is also possible to laminate the protective layer 33 on the lens layer 32 (St24).

It is possible to produce the chemical sensor 1 in this way. It should be noted that the method of producing the chemical sensor 1 is not limited the one described above, and a different producing method may be used.

### [Second embodiment]

A chemical sensor according to a second embodiment will be described. It should be noted that in this embodiment, description of the same configuration as those described in the first embodiment will be omitted. The chemical sensor according to this embodiment may include peripheral circuits and a light guide unit laminated on a substrate, as in the first embodiment. In addition, the chemical sensor according to this embodiment may be used as a chemical detection apparatus as in the first embodiment.

### [Structure of chemical sensor]

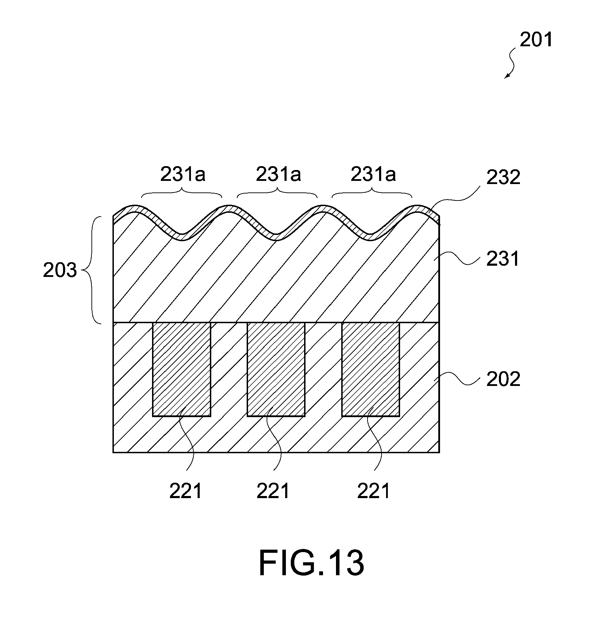

Fig. 13 is a schematic diagram showing a configuration of a chemical sensor 201 according to this embodiment. As shown in the figure, a light guide unit 203 is laminated on a substrate 202 on which light detection units 221 are formed, thereby forming the chemical sensor 201.

The configuration of the light detection unit 221 is the same as that in the first embodiment, i.e., the light detection unit 221 may be an impurity area that is formed by introducing an impurity on the substrate 2 being a semiconductor substrate. A pixel circuit (not shown) is connected to the light detection unit 221.

The light guide unit 203 includes a lens layer 231 and a protective layer 232. The lens layer 231 is laminated on the substrate 2, and the protective layer 232 is laminated on the lens layer 231.

The lens layer 231 provides a lens structure that supports a detection target object and collects light emitted from the detection target object on the light detection units 21 as in the first embodiment. Furthermore, the lens layer 231 according to this embodiment shields illumination light applied to the chemical sensor 201, and causes emitted light emitted from a detection target object to transmit therethough. Specifically, the lens layer 231 has also a function as a spectral filter. Because the lens layer 231 has also a function as a spectral filter, there is no need to provide a spectral filter layer separately and it is possible to lower the height of the light guide unit 203.

The lens layer 231 has the first refractive index as in the first embodiment, provides a concave lens structure 231a that supports a detection target object, and collects light emitted from the detection target object on the light detection unit 221. As shown in Fig. 13, a plurality of lens structures 231a may be formed, and each lens structure 231a may be formed to face the light detection unit 221.

The protective layer 232 covers the lens layer 231, and holds a detection target object on the surface thereof. The protective layer 232 may have liquid-repellent properties with respect to a detection target object-containing solution and has a function to contain a detection target object in each lens structure 231a as in the first embodiment. It does not necessarily need to provide the protective layer 232, and such a surface process that the surface of the lens layer 231 is made to have liquid-repellent properties with respect to a detection target object-containing solution may be applied to the surface of the lens layer 231 instead of the protective layer 232.

The chemical sensor 201 has the configuration described above. Also in the chemical sensor 201, a detection target object-containing solution supplied to the surface of the protective layer 232 is collected on each lens structure 231a by the shape of the lens structure 231a and the liquid-repellent properties of the protective layer 232 as in the first embodiment. Therefore, it is possible to cause a detection target object to be contained in each lens structure 231a. Furthermore, because the lens structure 231a functions as a lens, light emitted from a detection target object is collected on the light detection unit 221, and thus emitted light can be detected efficiently.

### [Method of producing chemical sensor]

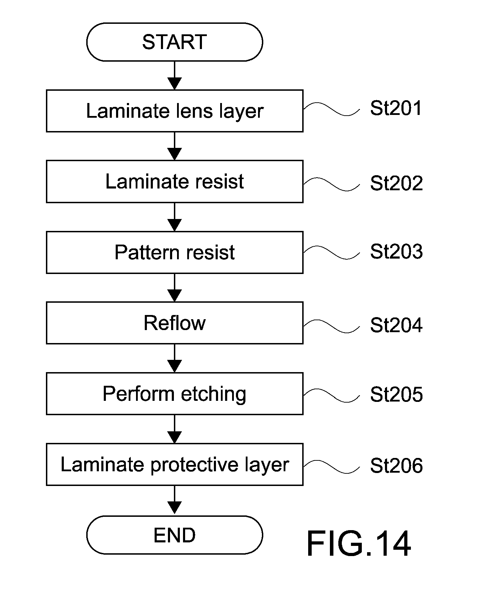

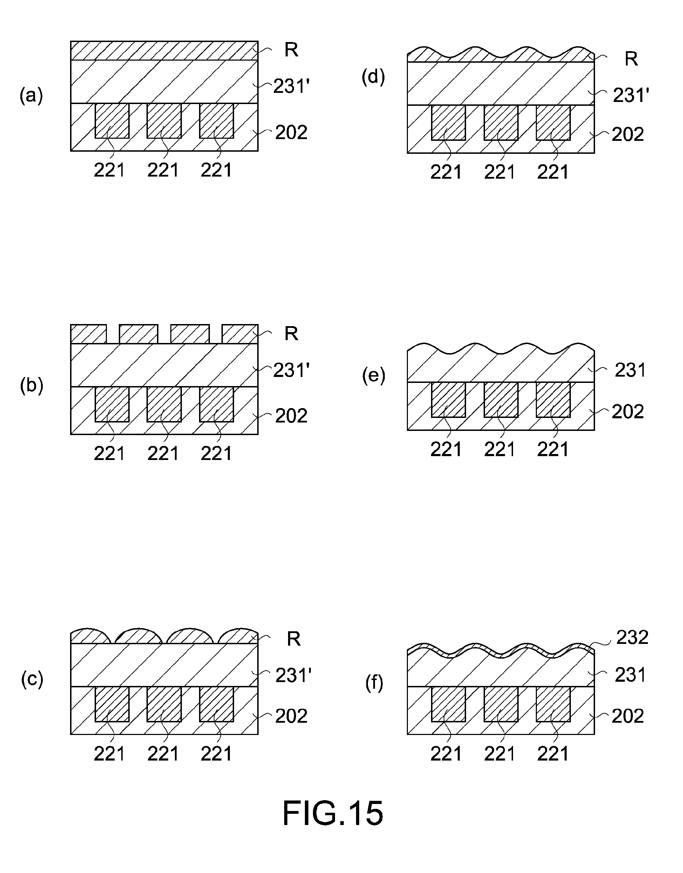

A method of producing the chemical sensor 201 will be described. Fig. 14 is a flowchart showing a method of producing the chemical sensor 201, and Figs. 15 are cross-sectional views showing the chemical sensor 201 at each producing stage.

On the substrate 202 on which the light detection units 221 are formed, a lens layer 231' is laminated (St201). It is possible to laminate the lens layer 231' by an arbitrary method. Next, as shown in Fig. 15(a), a resist R is laminated on the lens layer 231' (St202). The resist R may be formed of a thermoplastic material that can flow with viscosity by heating.

Next, as shown in Fig. 15(b), the resist R is patterned (St203). The patterning of the resist R can be performed by removing the resist R above each light detection unit 221 linearly as in the patterning of the lens layer in the first embodiment. By the patterning, the resist R is divided into a plurality of sections.

Next, as shown in Fig. 15(c), the patterned resist R is heated (reflowed) (St204). The resist R becomes fluid having viscosity by the heating. If the heating is continued, adjacent sections of the resist R are connected to each other as shown in Fig. 15(d). In this way, the resist R is formed in a concave shape.

Next, as shown in Fig. 15(e), the resist R is used to etch the lens layer 231' (St205). By the etching, the resist R is removed and the concave shape of the resist R is transferred to the lens layer 231'. The etching may be, for example, a dry etching. Accordingly, the concave lens structure 231a can be formed on the lens layer 231.

Next, as shown in Fig. 15(f), the protective layer 232 is laminated on the lens layer 231 (St206). It is possible to laminate the protective layer 232 by an arbitrary method. Moreover, the producing process may be finished without laminating the protective layer 232, and such a surface process that the lens layer 231 is made to have liquid-repellent properties with respect to a detection target object-containing solution may be applied to the lens layer 231 instead of the protective layer 232.

Furthermore, peripheral circuits are mounted on the substrate 202 as necessary, thereby producing the chemical sensor 201. It should be noted that these peripheral circuits may be mounted on the substrate 202 prior to the producing process. It is possible to produce the chemical sensor 201 in this way. It should be noted that the method of producing the chemical sensor 201 is not limited to the one described above, and a different producing method may be used.

The present technology is not limited only to the above-mentioned embodiments and various modifications can be made without departing from the gist of the present technology.

The chemical sensor according to the above-mentioned embodiments may include a light-shielding film for preventing emitted light from leaking from an adjacent lens structure. The light-shielding film may be formed by arranging a film formed of a material having high light-shielding properties so that the upper layer of each light detection unit is separated for each light detection unit.

It should be noted that the present technology may also take the following configurations.

(1) A chemical sensor, including:
   a substrate on which at least one light detection unit is formed;
   a lens layer that is laminated on the substrate and has optical transparency; and
   a lens structure formed on a surface of the lens layer opposite to the substrate in a concave shape toward a lamination direction.
(2) The chemical sensor according to (1) above, in which
   the lens structure faces the light detection unit.
(3) The chemical sensor according to (1) or (2) above, in which
   the light detection unit includes a plurality of light detection units, the plurality of light detection units are arranged on the substrate, the lens structure includes a plurality of lens structures, and the respective lens structures face the respective light detection units.
(4) The chemical sensor according to any one of (1) to (3) above, in which
   the lens layer has shielding properties with respect to a wavelength band of illumination light applied to the chemical sensor.
(5) The chemical sensor according to any one of (1) to (4) above, further including
   a spectral filter layer that is laminated between the substrate and the lens layer, and has shielding properties with respect to a wavelength band of illumination light applied to the chemical sensor.
(6) The chemical sensor according to any one of (1) to (5) above, in which
   the lens layer has a first refractive index and the spectral filter layer has a second refractive index that is larger than the first refractive index.
(7) The chemical sensor according to any one of (1) to (6) above, further including
   a protective layer that is laminated on the lens layer and has liquid-repellent properties with respect to a solution containing a detection target object.
(8) The chemical sensor according to any one of (1) to (7) above, in which
   the surface of the lens layer opposite to the substrate has liquid-repellent properties with respect to a solution containing a detection target object.
(9) The chemical sensor according to any one of (1) to (8) above, in which
   the lens structure is formed in a spherical lens shape.
(10) The chemical sensor according to any one of (1) to (9) above, in which
   the lens structure is formed in a cylindrical lens shape.
(11) A method of producing a chemical sensor, including:
   laminating a thermoplastic material on a substrate on which a plurality of light detection units are formed;
   patterning the thermoplastic material into a plurality of sections;
   connecting the adjacent sections of the thermoplastic material to each other by heating the thermoplastic material; and
   forming the thermoplastic material in a concave shape.
(12) The method of producing a chemical sensor according to (11) above, in which
   in the process of patterning the thermoplastic material, the thermoplastic material is patterned by removing the thermoplastic material above the light detection unit linearly.
(13) A chemical detection apparatus, including:
   a chemical sensor including
      a substrate on which at least one light detection unit is formed,
      a lens layer that is laminated on the substrate and has optical transparency, and
      a lens structure formed on a surface of the lens layer opposite to the substrate in a concave shape toward a lamination direction;
   an illumination light source that applies illumination light to the chemical sensor; and
   an image acquisition unit that acquires, based on an output from the light detection unit generated by emitted light that is generated from a detection target object contained in the lens structure by irradiation of the illumination light, an image of the emitted light. Description of Reference Numerals

- 1, 201: chemical sensor
- 2, 202: substrate
- 21, 221: light detection unit
- 31: spectral filter layer
- 32, 231: lens layer
- 32a, 231a: lens structure
- 33, 232: protective layer
- 100: chemical detection apparatus
- 101: illumination light source
- 102: image acquisition unit
- 201: chemical sensor
- 202: substrate
- 203: light guide unit

## Claims

1. A chemical sensor, comprising:
a substrate on which at least one light detection unit is formed;
a lens layer that is laminated on the substrate and has optical transparency; and
a lens structure formed on a surface of the lens layer opposite to the substrate in a concave shape toward a lamination direction.

2. The chemical sensor according to claim 1, wherein the lens structure faces the light detection unit.

3. The chemical sensor according to claim 2, wherein
the light detection unit includes a plurality of light detection units, the plurality of light detection units are arranged on the substrate, the lens structure includes a plurality of lens structures, and the respective lens structures face the respective light detection units.

4. The chemical sensor according to claim 1, wherein
the lens layer has shielding properties with respect to a wavelength band of illumination light applied to the chemical sensor.

5. The chemical sensor according to claim 1, further comprising
a spectral filter layer that is laminated between the substrate and the lens layer, and has shielding properties with respect to a wavelength band of illumination light applied to the chemical sensor.

6. The chemical sensor according to claim 5, wherein
the lens layer has a first refractive index and the spectral filter layer has a second refractive index that is larger than the first refractive index.

7. The chemical sensor according to claim 1, further comprising
a protective layer that is laminated on the lens layer and has liquid-repellent properties with respect to a solution containing a detection target object.

8. The chemical sensor according to claim 1, wherein
the surface of the lens layer opposite to the substrate has liquid-repellent properties with respect to a solution containing a detection target object.

9. The chemical sensor according to claim 1, wherein
the lens structure is formed in a spherical lens shape.

10. The chemical sensor according to claim 1, wherein
the lens structure is formed in a cylindrical lens shape.

11. A method of producing a chemical sensor, comprising:
laminating a thermoplastic material on a substrate on which a plurality of light detection units are formed;
patterning the thermoplastic material into a plurality of sections;
connecting the adjacent sections of the thermoplastic material to each other by heating the thermoplastic material; and
forming the thermoplastic material in a concave shape.

12. The method of producing a chemical sensor according to claim 11, wherein
in the process of patterning the thermoplastic material, the thermoplastic material is patterned by removing the thermoplastic material above the light detection unit linearly.

13. A chemical detection apparatus, comprising:
a chemical sensor including
a substrate on which at least one light detection unit is formed,
a lens layer that is laminated on the substrate and has optical transparency, and
a lens structure formed on a surface of the lens layer opposite to the substrate in a concave shape toward a lamination direction;
an illumination light source that applies illumination light to the chemical sensor; and
an image acquisition unit that acquires, based on an output from the light detection unit generated by emitted light that is generated from a detection target object contained in the lens structure by irradiation of the illumination light, an image of the emitted light.
